Europäisches Patentamt

European Patent Office

Office européen des brevets

⑱

(11) Publication number: **0 143 503
B1**

(12) **EUROPEAN PATENT SPECIFICATION**

(45) Date of publication of patent specification: **01.02.89**

(21) Application number: **84201719.6**

(22) Date of filing: **23.11.84**

(51) Int. Cl.⁴: **A 61 M 5/14**

(54) **Implantable hand-operable dispensers for fluid medicaments.**

(30) Priority: **23.11.83 NL 8304030
15.02.84 NL 8400489**

(43) Date of publication of application:
**05.06.85 Bulletin 85/23**

(45) Publication of the grant of the patent:
**01.02.89 Bulletin 89/05**

(84) Designated Contracting States:
**DE FR GB IT NL SE**

(56) References cited:
**EP-A-0 068 815
GB-A-2 028 275
US-A-3 827 439
US-A-4 013 074
US-A-4 152 098**

(73) Proprietor: **CORDIS CORPORATION
3901 Biscayne Boulevard
Miami Florida 33137 (US)**

(72) Inventor: **de Vries, Gerrit
Zijpendaal 47
NL-9301 WE Roden (NL)**
Inventor: **Leuveld, Jozef Gerhardus Maria
Diepswal 12
NL-9351 TA Leek (NL)**
Inventor: **Kleijn, Johan Willem
Zijpendaal 36
NL-9301 WG Roden (NL)**

(74) Representative: **Urbanus, Henricus Maria, Ir.
et al
c/o Vereenigde Octrooibureaux Nieuwe
Parklaan 107
NL-2587 BP 's-Gravenhage (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to an implantable, manually operable dispenser for a fluid medicament, comprising a body member having reservoir means for storing a fluid medicament, said reservoir means having a flexible wall that expands and collapses as fluid medicament flows respectively into and out of the reservoir means, a supply port assembly on said body member, said supply port assembly being in fluid-passing communication with said reservoir means, a connecting channel within said body member, said connecting channel having a section thereof that is in fluid-passing communication with said reservoir means and another section thereof that is in fluid-passing communication with a delivery tube outlet from said body member, propelling means within said connecting channel for advancing fluid medicament in a downstream direction through said connecting channel toward said delivery tube outlet and one-way blocking means within said connecting channel for permitting, in corporation with activation of said propelling means, controlled fluid flow therethrough toward said delivery tube outlet, said one-way blocking means also being for preventing fluid flow therethrough toward said delivery tube outlet, when said propelling means is not activated.

Such a dispenser or infusion apparatus is known from GB-A-2,028,275.

Sufferers from certain diseases, for example those patients that experience a great deal of pain, may benefit from the dosed administration of a medicament such as a sedative directly to or in close proximity of the pain center. In this connection, it should be borne in mind, for that matter, that pain centers may be located deep within the body, and the local administration of medication thereto typically involves a liquid medicament and requires means to transport the medicament to such an internal body location. Sometimes such administration involves a hypodermic syringe and needle and/or the services of a trained medical professional.

Said prior infusion apparatus now briefly includes a usual housing containing an infusate reservoir preferably in the form of a bellows capsule situated in a housing chamber filled with a two-phase fluid which tends to expand at body temperature and compress the bellows. The apparatus may be refilled and recharged periodically by injecting fresh infusate through a septum located in the housing wall. An outlet passage leads from the main bellows to a second chamber within the housing with the end of the passage inside the second chamber being shaped to function as a valve seat. A second passage having one end located in the second chamber, also shaped to function as a valve seat extends through the housing wall and is connected to a capillary tube whose opposite end communicates with a catheter which is placed at the selected infusion site within the patient's body. Both the first and second passages are valved. The apparatus also includes a third chamber which contains a second bellows capsule which is mounted such that the interior of the second capsule communicates with the second chamber. So the auxiliary capsule is basically a variable volume extension of the second chamber.

It is a feature of the prior apparatus that if there is a failure of the valve means, the patient under the worst circumstances may receive a total dose equal to the volumetric change in the auxiliary bellows capsule.

It is an object of the invention to provide a hand-operable dispenser for a fluid medicament which is safeguarded against accidental or any unintentional administration of the fluid medicament.

Another object of this invention is to provide an improved implantable medicament dispenser that permits the patient to safely and conveniently self-administer a medicament fluid, even one which is highly viscous.

Another object of this invention is to provide a self-administration fluid medicament dispenser that dispenses a unit dosage of the medicament in response to only deliberate and knowing actions by the person administering the medicament.

According to the invention there is provided an implantable, hand-operable dispenser of the type mentioned in the opening paragraph including another controlled-flow blocking means positioned within a segment of the connecting channel that is between said propelling means and said reservoir means.

The invention is further elucidated, by way of example, through two embodiments of the dispenser in the accompanying drawings, wherein:

Fig. 1 is a cross-sectional view through the preferred medicament dispenser according to this invention that requires serial activation of two distinct propelling assemblies in order to dispense fluid therefrom;

Fig. 2 is a cross-sectional view through another embodiment of the medicament dispenser according to this invention which dispenses the fluid by manipulating a single activation site;

Fig. 3 is a perspective view of the medicament dispenser shown in Figure 1; and

Figure 4 is a top plan view of the medicament dispenser shown in Figure 1.

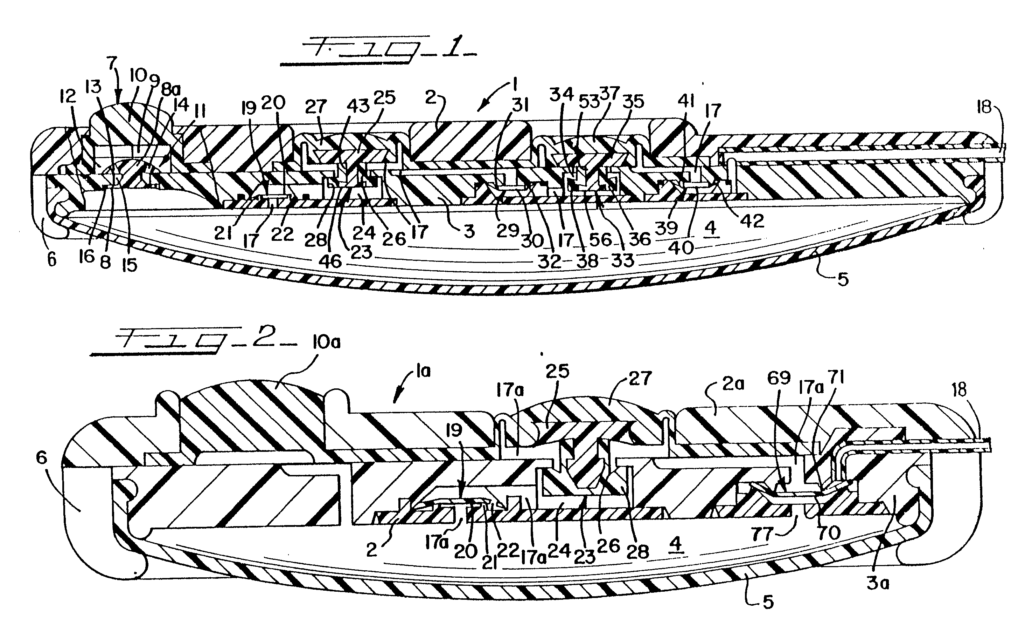

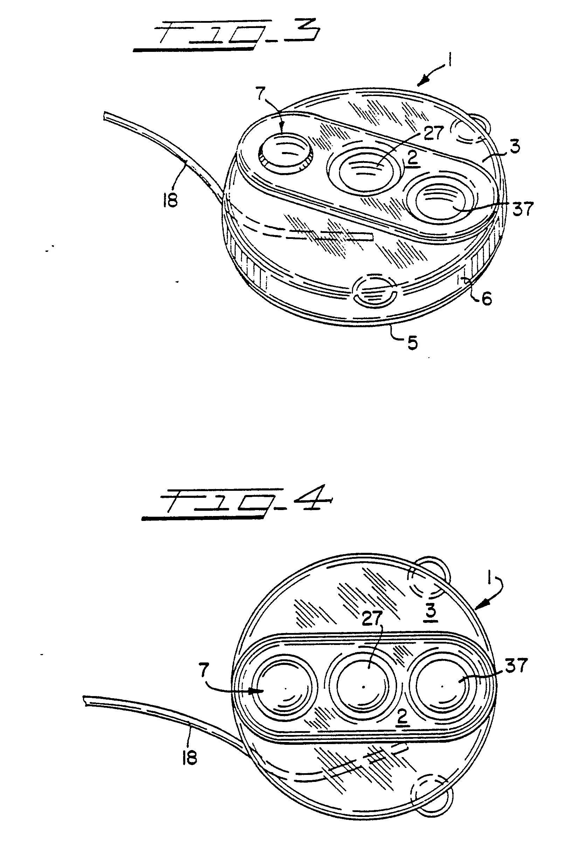

The dispensing means illustrated in Figures 1, 3 and 4, generally designated by reference numeral 1, has a casing which typically includes an upstanding component 2 and a body component 3, both of which are made of a relatively rigid, biocompatible polymeric material. Polyethyl sulfone is an exemplary biocompatible material that is suitable in this regard. Upstanding component 2 and body component 3 are provided as integrally molded units or are secured together and/or to each other by appropriate means such as ultrasonic sealing or structures which provide fixed connections such as adhesives or screws.

A reservoir 4 is partly defined by a yielding wall 5 that is secured to the body component 3 by suitable means such as the illustrated snap-on ring 6 which provides a tight-sealing assembly of the periphery of the yielding wall 5 to the body component 3. Reservoir 4 is further defined by the underside of the body component 3. Yielding wall 5 is structured and fabricated of a material such that it insures that the liquid contents of the reservoir substantially fill the reservoir 4 even as the liquid medicament confined thereby is dispensed out of the reservoir 4. In other words, as each unit dose of liquid medicament is withdrawn from the reservoir 4, the yielding wall 5 is displaced toward the body component 3 in order to maintain close confinement of the medicament remaining within the reservoir 4 so that the medicament therewithin is maintained under a substantially constant pressure and so that air pockets are not developed therewithin. Yielding wall 5 is, for example, suitably made of a silicone rubber material that is reinforced with polyester fibers.

The entire dispensing device is designed to be suitable for and to be operative under conditions that obtain when such a device is implanted under the skin of a patient. In this regard, a supply port assembly, generally designated by reference numeral 7, provides a pathway through which the liquid medicament can be inserted into the reservoir 4 from outside of the patient's body, typically by means of a hypodermic needle device. Supply port assembly 7 includes a generally mushroom-shaped shut-off valve, generally designated by reference numeral 11, a needle stop plate 9 of generally known construction, and a domed pad 10 that projects above the upstanding component 2 of the device 1 in order to assist in the accurate location thereof when the device is implanted within the patient. Supply port assembly 7 further includes a chamber 12 that is recessed within the domed pad 10. The needle stop plate 9 and an extending cap 8a of the mushroom-shaped shutoff valve 11 are both disposed within the chamber 12. Channels 13 and 14 are provided through the extending cap 8a of the mushroom-shaped shutoff valve 11, which channels 13 and 14 communicate the chamber 12 with an annular space 15 located between the extending cap 8a and an extending foot 8 of the shutoff valve 11. Preferably, the space 15 is countersunk to form a disk-shaped space 16 which receives the extending foot 8.

Mushroom-shaped shutoff valve 11 is sized and structured such that its extending foot 8, under at-rest conditions, is sealingly positioned within the disk-shaped space 16, so as to prevent passage of liquid across the shutoff valve 11, particularly between the chamber 12 and the reservoir 4. Reservoir 4 is filled by inserting the needle of a hypodermic syringe filled with medicament through the domed pad 10, thereby engaging the substantially rigid stop plate 9 (made, for example, of a polysulfone). The stop plate 9 in turn pushes on the extending cap 8a of the shutoff valve 11 which, because it is made of a resilient material such as a silicone rubber, is deformed and pushed inwardly until the extending foot 8 is pushed away from its engagement with the disk-shaped space 16. When the plunger of the hypodermic needle is depressed and the liquid medicament flows out of the needle, the liquid medicament flows, by way of a slit-shaped space adjacent to the side wall of the needle stop plate 9, into the chamber 12, through channels 13 and 14, through the spaces 15 and 16, and into the reservoir 4. After the hypodermic needle is withdrawn from the domed pad 10, the domed pad 10 seals itself automatically in accordance with well-known self-sealing port principles, and the needle stop plate 9 returns to its at-rest position, which permits the mushroom-shaped shutoff valve 11 likewise to return to its at-rest position and original shape in order to once again block the passage of liquid between the chamber 12 and the reservoir 4.

A connecting channel 17 having a plurality of channel segments traverses a path that ultimately provides fluid-passing communication between the reservoir 4 and a delivery tube 18. Preferably, delivery tube 18 is made of a material that incorporates a contrast medium, so that the guiding of the delivery tube 18 to the site of treatment can be monitored by well-known X-ray techniques. Silicone rubber tubes incorporating such a contrast medium are suitable in this regard.

With more particular reference to connecting channel 17, an initial channel segment thereof provides a passageway between the reservoir 4 and a one-way valve assembly, generally designated by reference numeral 19, which includes a diaphragm 20, typically made of a resilient material such as silicone rubber, which, when the device is at rest, is in sealing engagement with a collar 21. Diaphragm 20 includes an aperture 22 through which liquid medicament may pass when the diaphragm 20 is moved by appropriate liquid pressures out of full sealing engagement with the collar 21.

The next channel segment of connecting channel 17 provides a passageway to a recessed housing or chamber 24 within the body component 3, within which recessed chamber 24 is slidably mounted a portion of a blocking assembly, generally designated by reference numeral 23. Blocking assembly 23 includes a plunger assembly for selectively opening and closing the recessed housing 24 to a further channel segment of the connecting channel 17 which is downstream of the blocking assembly 23. The illustrated plunger assembly of the blocking assembly 23 includes a pushbutton 25 (for example made of a polysulfone), a resilient sealing sleeve 26 (for example made of a silicone rubber) and a resilient pad 27 (for example made of a silicone rubber) into which the top portion of the pushbutton 25 is imbedded. Resilient pad 27 is structured so that the plunger assembly is loaded or biased such that, when the device is at rest, a corner edge 28 of the sealing sleeve 26 engages a surface of the recessed chamber 24, thereby closing communication between the recessed chamber 24 and the segment of the connecting channel 17 which is present when the blocking assembly 23 is in its illustrated at-rest position. Preferably, the top surface of the resilient pad 27 is countersunk with respect to the outside surface of the upstanding casing component 2.

Another one-way valve assembly, generally designated by reference numeral 29, is interposed between the previously described segment of the connecting segment 17 and a further downstream segment thereof. One-way valve assembly 29, which is structured to be substantially the same as one-way valve assembly 19, includes a diaphragm 30 sealingly mounted onto a collar 31. Diaphragm 30 includes an aperture 32. Downstream of the further segment of the connecting channel 17 is another blocking assembly, generally designated by reference numeral 33, which blocking assembly 33 is similar to blocking assembly 23, except for one important difference. Blocking assembly 33 is biased or loaded, by the configuration and sizing of its resilient pad 37, in a generally downwardly oriented position at which the bottom or inside surface of its pushbutton 35 engages the body component 3 in order to preclude the formation of a channel segment therebetween.

A recessed chamber 34 within the body component 3 receives the lower portion of the pushbutton 35 and its sealing sleeve 36. When fluid pressure is suitably developed, the blocking assembly 33 moves upwardly until a corner edge 38" of the sealing sleeve 36 closes the recessed chamber 34, at which time a segment of the connecting channel 17 is formed between the upper surface of the body component 3 and the inside bottom surface of resilient pad 37 and the head of pushbutton 35. Thus formed connecting segment, when present, communicates with a terminal section of the connecting channel 17, which opens to another one-way valve assembly, generally designated by reference numeral 39. When open, one-way valve assembly 39 communicates with the delivery tube 18. One-way valve assembly 39 includes a diaphragm 40 in sealing engagement with a collar 41 when the device is at rest. Diaphragm 40 includes an aperture 42.

In Figure 1, the medicament dispensing device is shown in its at-rest or quiescent state at which it is in a non-dispensing mode. Each of the one-way valve assemblies 19, 29 and 39 are closed, and the blocking assembly 23 functions as a plunger in its uppermost or outwardly extending position, while the blocking assembly 33 functions as a plunger in its lowermost or inwardly directed position at which the corner edge 38 thereof is spaced from the body component 3. When it is desired to dispense a unit dosage of fluid medicament into and through the delivery tube 18, with fluid filled within the connecting channel 17, the user first depresses the blocking assembly 23, which propels fluid medicament that will raise or outwardly extend the blocking assembly 33. Dispensing is carried out when the thus outwardly extended blocking assembly 33 is depressed to return to its inwardly directed at-rest orientation which is the orientation shown in Figure 1. Thus, the blocking assembly 33 functions as a means for propelling the fluid through the terminal section of the connecting channel 17 and through the delivery tube 18.

With more particular reference to the steps by which liquid medicament passes through each segment of the connecting channel 17, the following sequence of events occurs. When the blocking assembly 23 is depressed, the pushbutton 25 and its sealing sleeve 26 move downwardly, thereby releasing the seal between the corner edge 28 and the recessed housing 24. Such depressing of the blocking assembly 23 causes the blocking assembly 23 to function as a plunger whereby fluid in the recessed housing 24 exerts pressure on the diaphragm 20 of the one-way valve assembly 19 to thereby safeguard the maintenance of the seal between the diaphragm 20 and its collar 21. At the same time, a metered quantity of liquid medicament is forced from the recessed housing or chamber 24 by movement of the pushbutton 25 and its sealing sleeve 26 into the recessed chamber 24, which displaces medicament generally upwardly through the blocking assembly 23 and to the next downstream segment of the connecting channel 17 toward the one-way valve assembly 29.

This downstream displacement of fluid and simultaneous exertion of pressure on the diaphragm 20 of the one-way valve assembly 19 in order to maintain same in a closed state is facilitated by providing a spacing between bottom surface 46 of the blocking assembly 23, which is achieved in part by appropriately sizing the length of stem 43 of the pushbutton 25. This downstreamwardly directed pressure is imparted to liquid medicament already in the downstream section of connecting channel 17, which in turn exerts pressure on the diaphragm 30 of the one-way valve assembly 29, this in turn causing a lifting of the diaphragm 30 off of its collar 31, whereby the liquid medicament within this downstream section of the connecting channel 17 flows through aperture 32, then through the further downstream segment of the connecting channel 17, and into the recessed chamber 34 of the blocking assembly 33.

At this stage, the liquid medicament flowing through aperture 32 exerts a hydraulic force on the bottom surface 56 of the blocking assembly 33, which in turn raises the blocking assembly 33. This activity is assisted by providing spacing between the bottom surface 56 of the blocking assembly 33 and the opposing wall of the recessed chamber 34, which is accomplished by appropriately sizing stem 53 of the pushbutton 35. Blocking assembly 33 is thereby displaced upwardly or outwardly through a distance that is substantially equal to that through which the blocking assembly 23 is displaced inwardly when pressed by the user, such being achieved by providing generally equal volumeric respective sizings of the blocking assembly 23 and of the blocking assembly 33, their respective recessed chambers 24 and 34, so that the entire incremental volume of liquid medicament that is displaced from chamber 24 by depressing the blocking assembly 23 is received in the recessed chamber 34, at which time the recessed chamber 34 is sealed when the corner edge 38 engages the upper or outwardly directed surface of the recessed chamber 34.

If appropriate safeguards are not taken, the outward or upward displacement of fluid medicament through the blocking assembly 33 could result in opening of the one-way valve assembly 39, which would permit a somewhat uncontrolled and non-incremental volume of liquid medicament to flow through the aperture 42 and into the delivery tube 18. Although this undesirable result should cease when the corner edge 38 seals off the recessed chamber 34, this possibility is preferably prevented by designing the one-way valve assembly 39 such that the pressure that is required to lift the diaphragm 40 off of its collar 41 is greater than the pressure required to displace the blocking assembly 33 outwardly or upwardly. Moreover, this pressure required to open the one-way valve assembly 39 is less than that which is developed when the blocking assembly 33 is depressed. In other words, the hydraulic pressure generated by depressing the blocking assembly 33 is of such a high value that it lifts the diaphragm 40 off of its collar 41 to permit an incremental unitary dosage amount of liquid medicament to flow through the aperture 42, into the delivery tube 18 and to the site where the fluid medicament is to be administered.

With further reference to the blocking assembly 23, when same is depressed, it moves until the pushbutton 25 engages the body component 3. As soon as the blocking assembly 23 is released, the pushbutton 25 moves upwardly or outwardly to return to its at-rest position by virtue of the loaded or biased attributes of the resilient pad 27 which had been deformed during depression of the blocking assembly 23. As a result of this return to the upward or outward orientation, the spacing between the bottom surface 46 and the opposing wall of the recessed chamber 24 increases to thereby lift the diaphragm 20 until the moment when the corner edge 28 has again reached the position shown in Figure 1 and recessed chamber 24 is again closed. During the time that the diaphragm 20 is lifted off of its collar 21, medicament flows from the reservoir 4 and through the aperture 22 of the one-way valve assembly 19, this flow being assisted by the collapsibility and flexibility of the yielding wall 5 such that the volume of the reservoir 4 is reduced as the yielding wall 5 collapses when the medicament passes through the one-way valve assembly 19 while the device is implanted and in contact with the patient.

From the preceding, it is clear that the dosage of a metered quantity of a liquid medicament will be dispensed out of the delivery tube 18 only after the device according to this embodiment is subjected to two distinct manipulations. First, blocking assembly 23 must be depressed, which results in the raising or outward extension of the blocking assembly 33. Then, in order for the medicament to be dispensed, a second distinct step must performed, which second step is the depression of the blocking assembly 33. This provides a built-in security factor which substantially eliminates the possibility of an inadvertent dosage of medicament being dispensed through the delivery tube 18.

With reference to the embodiment illustrated in Figure 2, this device 1a includes only a single blocking assembly 23 which performs a dual function of providing blocking attributes while also functioning as means for propelling liquid medicament through the delivery tube 18, when such delivery is desired. Device 1a also includes a domed pad 10a projecting above upstanding component 2a providing a relatively simple construction for filling the reservoir 4 by passing a hypodermic needle theretthrough. Also included is the one-way valve assembly 19 and a further one-way valve assembly 69 which does not have an aperture through its diaphragm, valve assemblies 19 and 69 being within body component 3a.

The at-rest or quiescent state of device 1a is illustrated in Figure 2, this being the position of all components of the device 1a between the times at which medicament dispensing is desired. More particularly, the blocking assembly 23 is in its upward or outwardly extending position at which the corner edge 28 of the sealing sleeve 26 sealingly engages the outwardly extending wall of the recessed chamber 24. When it is desired to supply a dose of medicament through the delivery tube 18, pressure is exerted on the pushbutton 25 through the resilient pad 27 of the blocking assembly 23 in order to depress same and remove the sealing engagement between the corner edge 28 and the recessed chamber 24. Because liquid medicament is included within the section of the connecting channel 17a which is between the blocking assembly 23 and the one-way valve assembly 19, the diaphragm 20 is forced onto its collar 21 in order to prevent passage of liquid medicament through its aperture 22. On the other hand, this same depression of the blocking assembly 23 exerts pressure on the section of the connecting channel 17a between the blocking assembly 23 and the one-way valve assembly 69 which results in the lifting of its diaphragm 70 off of its collar 71, whereby the liquid medicament flows through the thus formed opening between the collar 71 and the diaphragm 70 and into the delivery tube 18.

When the depressed blocking assembly 23 is released, it will return to its upward or outwardly extending orientation as illustrated in Figure 2, which upward movement creates a lowered pressure for fluid sealed within the blocking assembly 23 so as to cause a slight reverse flow of fluid medicament which results in a sealing of the diaphragm 70 against its collar 71. Simultaneously, this return movement of the blocking assembly 23 to its upward or outwardly extending orientation opens an additional volume within the recessed chamber 24, in response to which liquid medicament flows, thereby lifting the diaphragm 20 off of its collar 21 and flowing liquid medicament through the aperture 22 from the reservoir 4 in order to thereby refill the recessed chamber 24.

A channel 77 is provided for liquid pressure communication between the reservoir 4 and the diaphragm 70 of the one-way valve assembly 69. Aperture free diaphragm 70 is structured and positioned so that liquid medicament cannot flow therethrough from the channel 77. An increase in pressure caused by an increase in pressure on the outside surface of the reservoir 4 will enhance the seating of the diaphragm 70 onto its collar 71 in order to ensure that, if such outside pressure is increased onto the implanted reservoir 4, such increase will not cause medicament to be dispensed through the delivery tube 18.

## Claims

1. An implantable, hand-operable dispenser for a fluid medicament, comprising:

(a) a body member (1) having reservoir means (4) for storing a fluid medicament, said reservoir means (4) having a flexible wall (5) that expands and collapses as fluid medicament flows respectively into and out of the reservoir means (4);

(b) a supply port assembly (7) on said body member (1), said supply port assembly (7) being in fluid-passing communication with said reservoir means (4);

(c) a connecting channel (17) within said body member (1), said connecting channel (17) having a section thereof that is in fluid-passing communication with said reservoir means (4) and another section thereof that is in fluid-passing communication with a delivery tube outlet (18) from said body member;

(d) propelling means (23) within said connecting channel (17) for advancing fluid medicament in a downstream direction through said connecting channel (17) toward said delivery tube outlet (18); and

(e) one-way blocking means (69) within said connecting channel (17) for permitting, in cooperation with activation of said propelling means (23), controlled fluid flow therethrough toward said delivery tube outlet (18), said one-way blocking means (69) also being for preventing fluid flow therethrough toward said delivery tube outlet (18) when said propelling means (23) is not activated, characterized by further including another controlled-flow blocking means (19) positioned within a segment of the connecting channel (17) that is between said propelling means (23) and said reservoir means (4).

2. The implantable dispenser according to claim 1, characterized in that the body member (1) includes a plurality of said propelling means (23) for metering and displacing substantially equal quantities of liquid medicament, and another blocking means is provided between said plurality of propelling means (23).

3. The implantable dispenser according to claim 1, characterized in that a plurality of blocking means (19, 69) are provided so as to be both upstream and downstream of said propelling means (23).

4. The implantable dispenser according to claim 3, characterized by further including a plurality of said propelling means (23, 33) having blocking means (19, 29, 39, 69) both upstream and downstream thereof.

5. The implantable dispenser according to claim 4, characterized by further including three blocking means (19, 29, 39) and two propelling means (23, 33) that are alternately located with respect to each other within the connecting channel (17).

6. The implantable dispenser according to claim 1, characterized in that the propelling means (33, 23) includes sealing means (36, 26) for blocking the flow of fluid therethrough when said propelling means (33) is at its inwardly directed, depressed position and when said propelling means (23) is at its outwardly directed, extended position.

7. The implantable dispenser according to claim 1, characterized in that said blocking means (19, 29, 39, 69) includes a diaphragm (20, 30, 40, 70) that sealingly engages a collar (21, 31, 41, 71) of the body member (1), and wherein said propelling means (23, 33) includes plunger means for reciprocatingly opening and closing a chamber (24, 34) of the body member (1) through which said plunger means is mounted.

8. The implantable dispenser according to claim 5, characterized in that said blocking means (19, 29, 39) includes a diaphragm (20, 30, 40) that sealingly engages a collar (21, 31, 41) of the body member (1), and wherein said diaphragm has an aperture (22, 32, 42) therethrough.

9. The implantable dispenser according to claim 1, characterized in that said propelling means (23) includes a pushbutton (25) having a stem (43) extending into a chamber (24) of the body member (1), and resilient means (26) mounted to said stem (43) for sealingly engaging and closing said chamber (24) when said pushbutton (25) is at its outwardly directed, extended position.

10. The implantable dispenser according to claim 9, characterized in that said propelling means (23) further includes a resilient pad (27) to which said pushbutton (25) is mounted, said resilient pad (27) providing means for biasing said propelling means (23) to said sealing engagement and closing of said chamber (24).

11. The implantable dispenser according to claim 2, characterized in that the blocking means (39) closest to said outlet (18) from the body member (1) is structured for opening at a pressure greater than pressure needed for outwardly moving the propelling means (33) most closely spaced therefrom.

12. The implantable dispenser according to claim 1, characterized in that said supply port assembly (7) includes one-way means for shutting off flow between said supply port assembly (7) and said reservoir means (4).

13. The implantable dispenser according to claim 12, characterized in that said one-way means includes a resilient mushroom-shaped member (11) mounted within a passageway space through said body member (1), said resilient mushroom-shaped member (11) including an extending foot (8) partially defining said reservoir means and including an extending cap (8a) opposite of said extending foot (8), said extending cap (8a) having a channel (13, 14) therethrough.

14. The implantable dispenser according to claim 13, characterized in that said supply port assembly (7) further includes a needle stop plate (9) movable toward and away from said extending cap (8a) of the mushroom-shaped member (11), and a domed pad (10) covers said needle stop plate (9).

15. The implantable dispenser according to claim 3, characterized in that said blocking means (69) downstream of the propelling means (23) includes an imperforate diaphragm (70) that sealingly engages a collar (71) of the body member (1), and wherein means (77) are provided for transmitting fluid pressure within said reservoir means (4) to said imperforate diaphragm (70) and for sealingly engaging said imperforate diaphragm (70) onto its said collar (71).

16. The implantable dispenser according to claim 2, characterized in that the propelling means (33) that is most downstream includes resilient means (37) mounting a pushbutton (35) of the propelling means (33) to said body member (1), said resilient means biasing said pushbutton in a depressed orientation, and wherein upstream propelling means (23) includes resilient means (27) mounting a pushbutton (25) of the propelling means (23) to said body member (1), said resilient means (27) biasing said pushbutton (25) in an outwardly extended orientation.

17. The implantable dispenser according to claim 1, characterized in that said blocking means (19) between the propelling means (23) and the reservoir means (4) includes a diaphragm (20) that sealingly engages a collar (21) of the body member (1), and wherein said diaphragm (20) has an aperture (22) therethrough.

18. The implantable dispenser according to claim 1, characterized in that said propelling means (23, 33) includes plunger means for reciprocatingly opening and closing a chamber (24, 34) of the body member in communication with said connecting channel (17).

19. The implantable dispenser according to claim 16, characterized in that said downstream propelling means (33) and said upstream propelling means (23) are in fluid-passing communication through a segment of said connecting channel (17), whereby depression of said upstream propelling means (23) outwardly extends said downstream propelling means (33).

**Patentansprüche**

1. Implantierbare, handbedienbare Abgabeeinrichtung für ein flüssiges Medikament mit

(a) einem Gehäusekörper (1) mit einem Vorratsbehälter (4) zur Speicherung eines flüssigen Medikaments, der eine flexible Wandung (5) hat, die sich ausdehnt bzw. zusammenzieht, wenn flüssiges Medikament in den bzw. aus dem Vorratsbehälter (4) fließt,

(b) einer an dem Gehäusekörper (1) angeordneten Versorgungseinheit, die mit dem Vorratsbehälter in flüssigkeitsleitender Verbindung steht,

(c) einem in dem Gehäusekörper (1) angeordneten Verbindungskanal (17) mit einem Abschnitt, der mit dem Vorratsbehälter (4) in flüssigkeitsleitender Verbindung steht, und einem weiteren Abschnitt, der mit einem aus dem Gehäusekörper (1) herausführenden Ausgabetubus (18) in flüssigkeitsleitender Verbindung steht,

(d) einer in dem genannten Verbindungskanal (17) angeordneten Antriebseinrichtung (23) zum Vorbringen von flüssigem Medikament durch den Verbindungskanal (17) in stromabwärtiger Richtung zu dem Ausgabetubus (18) und

(e) einer in dem Verbindungskanal (17) angeordneten, in nur einer Richtung wirksamen Blockierungseinrichtung (6), die bei Aktivierung der Antriebseinrichtung (23) einen gesteuerten Flüssigkeitsdurchfluß durch den Verbindungskanal (17) zu dem Ausgabetubus (18) ermöglicht und einen Flüssigkeitsdurchfluß durch den Verbindungskanal (17) zu dem Ausgabetubus (18) verhindert, wenn die Antriebseinrichtung (23) nicht aktiviert ist,

dadurch gekennzeichnet,

daß ferner in einem zwischen der Antriebseinrichtung (23) und dem Vorratsbehälter (4) liegenden Abschnitt des Verbindungskanals (17) eine weitere gesteuerte Blockierungseinrichtung (19) angeordnet ist.

2. Implantierbare Abgabeeinrichtung nach Anspruch 1, dadurch gekennzeichnet, daß der Gehäusekörper (1) eine Mehrzahl der genannten Antriebseinrichtungen (23) aufweist, die ein Abmessen und Verschieben von im wesentlichen gleichen Mengen flüssigen Medikaments ermöglichen, und daß zwischen dieser Mehrzahl von Antriebseinrichtungen (23) eine weitere Blockierungseinrichtung vorgesehen ist.

3. Implantierbare Abgabeeinrichtung nach Anspruch 1, dadurch gekennzeichnet, daß eine Mehrzahl von Blockierungseinrichtungen (19, 69) vorgesehen ist, die stromaufwärts und stromabwärts der Antriebseinrichtung (23) angeordnet sind.

4. Implantierbare Abgabeeinrichtung nach Anspruch 3, dadurch gekennzeichnet, daß eine Mehrzahl der genannten Antriebseinrichtungen (23, 33) vorgesehen ist, denen sowohl auf der stromaufwärtigen als auch auf der stromabwärtigen Seite Blockierungseinrichtungen (19, 29, 39, 69) zugeordnet sind.

5. Implantierbare Abgabeeinrichtung nach Anspruch 4, dadurch gekennzeichnet, daß drei Blockierungseinrichtungen (19, 29, 39) und zwei Antriebseinrichtungen (23, 33) vorgesehen sind, die miteinander abwechselnd in dem Verbindungskanal (17) angeordnet sind.

6. Implantierbare Abgabeeinrichtung nach Anspruch 1, dadurch gekennzeichnet, daß die Antriebseinrichtung (33, 23) Dichtungsmittel (36, 26) enthält, die den Flüssigkeitsdurchfluß blockieren, wenn die Antriebseinrichtung (33) nach innen eingedrückt ist und die Antriebseinrichtung (23) ausgefahren ist.

7. Implantierbare Abgabeeinrichtung nach Anspruch 1, dadurch gekennzeichnet, daß die Blockierungseinrichtung (19, 29, 39, 69) eine Membran (20, 30, 40, 70) aufweist, die unter Abdichtung an einem Kragen (21, 31, 41, 71) des Gehäusekörpers (1) anliegt, und daß die Antriebseinrichtung (23, 33) ein Kolbenelement aufweist, das zum abwechselnden Öffnen und Schließen einer Kammer (24, 34) des Gehäusekörpers (1) dient, in der das Kolbenelement montiert ist.

8. Implantierbare Abgabeeinrichtung nach Anspruch 5, dadurch gekennzeichnet, daß die Blockierungseinrichtung (19, 29, 39, 39) eine Membran (20, 30, 40, 70) aufweist, die unter Abdichtung an einem Kragen (21, 31, 41) des Gehäusekörpers (1) anliegt, und daß diese Membran eine durchgehende Öffnung (22, 32, 42) aufweist.

9. Implantierbare Abgabeeinrichtung nach Anspruch 1, dadurch gekennzeichnet, daß die Antriebseinrichtung (23) eine Drucktaste (25) mit einem in eine Kammer (24) des Gehäusekörpers (1) ragenden Schaft (43) umfaßt sowie an diesem Schaft (43) angeordnete elastische Mittel (26) zum Abdichten und Schliessen der Kammer (24), wenn die Drucktaste (25) ausgefahren ist.

10. Implantierbare Abgabeeinrichtung nach Anspruch 9, dadurch gekennzeichnet, daß die Antriebseinrichtung (23) ferner ein elastisches Kissen (27) umfaßt, an dem die Drucktaste (25) montiert ist und das ein Mittel zum Vorspannen der Antriebseinrichtung (23) in die das Abdichten und Schließen der Kammer (24) bewirkende Position darstellt.

11. Implantierbare Abgabeeinrichtung nach Anspruch 2, dadurch gekennzeichnet, daß die Blockierungseinrichtung (39), die dem aus dem Gehäusekörper (1) herausführenden Ausgabetubus (18) am nächsten liegt, so konstruiert ist, daß sie bei einem Druck öffnet, der größer ist als der Druck, der erforderlich ist, um die ihr am nächsten liegenden Antriebseinrichtung (33) nach außen zu bewegen.

12. Implantierbare Abgabeeinrichtung nach Anspruch 1, dadurch gekennzeichnet, daß die Versorgungseinheit (7) in nur einer Richtung durchlässige Mittel zur Unterbrechung des Flüssigkeitsdurchgangs zwischen der Versorgungseinheit (7) und dem Vorratsbehälter (4) aufweist.

13. Implantierbare Abgabeeinrichtung nach Anspruch 12, dadurch gekennzeichnet, daß die in nur einer Richtung durchlässigen Mittel ein elastisches pilzförmiges Element (11) umfassen, das in einem durch den Gehäusekörper (1) führenden Durchgangsraum montiert ist und einen einen Teil der Begrenzung des Vorratsbehälters bildenden, erweiterten Fuß (8) sowie auf der diesem Fuß (8) entgegengesetzten Seite einenmit einem Durchgangskanal (13, 14) versehenen, erweiterten Kopf (8a) besitzt.

14. Implantierbare Abgabeeinrichtung nach Anspruch 13, dadurch gekennzeichnet, daß die Versorgungseinheit (7) außerdem eine Nadelanschlagplatte (9) besitzt, die auf den erweiterten Kopf (8a) des pilzförmigen Elements (11) zu und von diesem weg bewegbar ist, und daß diese Nadelanschlagplatte (9) von einem kuppelförmigens Kissen (10) abgedeckt ist.

15. Implantierbare Abgabeeinrichtung nach Anspruch 3, dadurch gekennzeichnet, daß die stromabwärts der Antriebseinrichtung (23) liegende Blockierungseinrichtung (69) eine unperforierte Membran (70) aufweist, die unter Abdichtung an einem Kragen (71) des Gehäusekörpers (1) anliegt, und daß Mittel (77) vorgesehen sind, die den in dem Vorratsbehälter (4) herrschenden Flüssigkeitsdruck auf diese unperforierte Membran (70) übertragen und diese unter Abdichtung an ihrem Kragen (71) zur Anlage bringen.

16. Implantierbare Abgabeeinrichtung nach Anspruch 2, dadurch gekennzeichnet, daß die am weitesten stromabwärts liegende Antriebseinrichtung (33) elastische Mittel (37) zum Montieren einer Drucktaste (35) der Antriebseinrichtung (33) an dem Gehäusekörper (1) aufweist, die die Drucktaste (35) in Druckrichtung vorspannen und daß eine stromaufwärts liegende Antriebseinrichtung (23) elastische Mittel (27) zum Montieren einer Drucktaste (25) der Antriebseinrichtung (23) an dem Gehäusekörper (1) aufweist, die die Drucktaste (25) in Ausdehnungrichtung nach außen vorspannen.

17. Implantierbare Abgabeeinrichtung nach Anspruch 1, dadurch gekennzeichnet, daß die zwischen der Antriebseinrichtung (23) und dem Vorratsbehälter (4) angeordnete Blockierungseinrichtung (19) eine Membran (20) umfaßt, die unter Abdichtung an einem Kragen (21) des Gehäusekörpers (1) anliegt und eine durchgehende Öffnung (22) aufweist.

18. Implantierbare Abgabeeinrichtung nach Anspruch 1, dadurch gekennzeichnet, daß die Antriebseinrichtung (23, 33) ein Kolbenelement für das abwechselnde Öffnen und Schließen einer mit dem Verbindungskanal (17) verbundenen Kammer (24, 34) aufweist.

19. Implantierbare Abgabeeinrichtung nach Anspruch 16, dadurch gekennzeichnet, daß die stromabwärts liegende Antriebseinrichtung (33) und die stromaufwärts liegende Antriebseinrichtung (23) über einen Abschnitt des Verbindungskanals (17) flüssigkeitsleitend miteinander verbunden sind, so daß das Niederdrücken der stromaufwärts liegenden Antriebseinrichtung (23) ein Ausdehnen der stromabwärts liegenden Antriebseinrichtung (33) zur Folge hat.

**Revendications**

1. Distributeur implantable, à commande manuelle, pour un médicament fluide, comprenant:

(a) un élément de corps (1) ayant un réservoir (4) pour emmagasiner un médicament fluide, ledit réservoir (4) comportant une paroi flexible (5) qui s'expanse et s'affaisse pendant qu'un médicament fluide, respectivement, pénètre dans le réservoir (4) et en sort en s'écoulant:

(b) un ensemble (7) à orifice d'alimentation situé sur ledit élément de corps (1), ledit ensemble (7) à orifice d'alimentation étant en communication de transmission de fluide avec ledit réservoir (4);

(c) un canal (17) de raccordement à l'intérieur dudit élément de corps (1), ledit canal (17) de raccordement ayant un tronçon qui est en communication de transmission de fluide avec ledit réservoir (4) et un autre tronçon qui est en communication de transmission de fluide avec une sortie (18) de tube de distribution provenant dudit élément de corps;

(d) des moyens de propulsion (23) à l'intérieur dudit canal (17) de raccordement, destinés à faire avancer un médicament fluide vers l'aval dans ledit canal (17) de raccordement vers ladite sortie (18) du tube de distribution; et

(e) des moyens (69) d'arrêt unidirectionnel à l'intérieur dudit canal (17) de raccordement, destinés à permettre, en coopération avec une activation desdits moyens de propulsion (23), un écoulement commandé du fluide à travers eux, vers ladite sortie (18) du tube de distribution, lesdits moyens d'arrêt unidirectionnel (69) étant également destinés à empêcher le fluide de s'écouler à travers eux vers ladite sortie (18) du tube de distribution lorsque lesdits moyens de propulsion (23) ne sont pas actionnés,

caractérisé en ce qu'il comporte en outre d'autres moyens (19) d'arrêt d'écoulement commandés disposés à l'intérieur d'un segment du canal (17) de raccordement qui est situé entre lesdits moyens de propulsion (23) et ledit réservoir (4).

2. Distributeur implantable selon la revendication 1, caractérisé en ce que l'élément de corps (1) comprend plusieurs desdits moyens de propulsion (23) pour doser et déplacer des quantités sensiblement égales de médicament liquide, et d'autres moyens d'arrêt sont prévus entre lesdits plusieurs moyens de propulsion (23).

3. Distributeur implantable selon la revendication 1, caractérisé en ce que plusieurs moyens d'arrêt (19, 69) sont prévus de façon à se trouver à la fois en amont et en aval desdits moyens de propulsion (23).

4. Distributeur implantable selon la revendication 3, caractérisé en ce qu'il comporte en outre plusieurs desdits moyens de propulsion (23, 33) comportant des moyens d'arrêt (19, 29, 39, 69) à la fois en amont et en aval d'eux.

5. Distributeur implantable selon la revendication 4, caractérisé en ce qu'il comporte en outre trois moyens d'arrêt (19, 29, 39) et deux moyens de propulsion (23, 33) qui sont disposés en alternance les uns par rapport aux autres à l'intérieur du canal (17) de raccordement.

6. Distributeur implantable selon la revendication 1, caractérisé en ce que les moyens de propulsion (23, 33) comprennent des moyens d'obturation étanches (36, 26) destinés à arrêter l'écoulement du fluide à travers eux lorsque lesdits moyens de propulsion (33) sont dans leur position d'enfoncement, en direction de l'intérieur, et lorsque lesdits moyens de propulsion (23) sont dans leur position d'extension, en direction de l'extérieur.

7. Distributeur implantable selon la revendication 1, caractérisé en ce que lesdits moyens d'arrêt (19, 29, 39, 69) comprennent un diaphragme (20, 30, 40, 70) qui porte de manière étanche contre une collerette (21, 31, 41, 71) de l'élément de corps (1), et dans lequel lesdits moyens de propulsion (23, 33) comprennent un moyen à plongeur destiné à ouvrir et fermer par un mouvement alternatif une chambre (24, 34) de l'élément de corps (1) dans laquelle ledit moyen à plongeur est monté.

8. Distributeur implantable selon la revendication 5, caractérisé en ce que lesdits moyens d'arrêt (19, 29, 39) comprennent un diaphragme (20, 30, 40) qui porte de manière étanche contre une collerette (21, 31, 41) de l'élément de corps (1), et dans lequel ledit diaphragme est traversé d'une ouverture (22, 32, 42).

9. Distributeur implantable selon la revendication 1, caractérisé en ce que lesdits moyens de propulsion (23) comprennent un bouton-poussoir (25) ayant une tige (43) qui pénètre dans une chambre (24) de l'élément de corps (1), et un moyen élastique (26) monté sur ladite tige (43) pour porter de façon étanche contre ladite chambre (24) et la fermer lorsque ledit bouton-poussoir (25) est dans sa position d'extension, en direction de l'extérieur.

10. Distributeur implantable selon la revendication 9, caractérisé en ce que lesdits moyens de propulsion (23) comprennent en outre un tampon élastique (27) sur lequel ledit bouton-poussoir (25) est monté, ledit tampon élastique (27) constituant un moyen pour rappeler lesdits moyens de propulsion (23) vers ledit contact étanche avec ladite chambre (24) et fermer celle-ci.

11. Distributeur implantable selon la revendication 2, caractérisé en ce que les moyens d'arrêt (39) les plus proches de ladite sortie (18) de l'élément de corps (1) ont une structure leur permettant de s'ouvrir sous une pression supérieure à la pression nécessaire pour déplacer vers l'extérieur les moyens de propulsion (33) qui en sont les plus proches.

12. Distributeur implantable selon la revendication 1, caractérisé en ce que ledit ensemble (7) à orifice d'alimentation comprend un moyen unidirectionnel destiné à arrêter l'écoulement entre ledit ensemble (7) à orifice d'alimentation et ledit réservoir (4).

13. Distributeur implantable selon la revendication 12, caractérisé en ce que ledit moyen unidirectionnel comprend un élément élastique (11) en forme de champignon monté à l'intérieur d'un espace de passage à travers ledit élément de corps (1), ledit élément élastique (11) en forme de champignon comprenant un pied étendu (8) qui définit partiellement ledit réservoir et comprenant un chapeau étendu (8a) opposé audit pied étendu (8), ledit chapeau étendu (8a) étant traversé par un canal (13, 14).

14. Distributeur implantable selon la revendication 13, caractérisé en ce que ledit ensemble (7) à orifice d'alimentation comprend en outre une plaque (9) de butée d'aiguille pouvant se rapprocher et s'éloigner dudit chapeau étendu (8a) de l'élément (11) en forme de champignon, et un tampon (10) en forme de dôme recouvre ladite plaque (9) de butée d'aiguille.

15. Distributeur implantable selon la revendication 3, caractérisé en ce que lesdits moyens d'arrêt (69) en aval des moyens de propulsion (23) comprennent un diaphragme non perforé (70) qui porte de façon étanche contre une collerette (71) de l'élément de corps (1), et dans lequel des moyens (77) sont prévus pour transmettre la pression d'un fluide, régnant à l'intérieur dudit réservoir (4), audit diaphragme non perforé (70) et pour faire porter de façon étanche ledit diaphragme perforé (70) sur ladite collerette (71).

16. Distributeur implantable selon la revendication 2, caractérisé en ce que les moyens de propulsion (33) situés le plus en aval comprennent un moyen élastique (37) à l'aide duquel le bouton-poussoir (35) des moyens de propulsion (33) est monté sur ledit élément de corps (1), ledit moyen élastique rappelant ledit bouton-poussoir dans une orientation enfoncée, et dans lequel des moyens de propulsion (23) d'amont comprennent un moyen élastique (27) à l'aide duquel un bouton-poussoir (25) des moyens de propulsion (23) est monté sur ledit élément de corps (1), ledit moyen élastique (27) rappelant ledit bouton-poussoir (25) dans une orientation en extension vers l'extérieur.

17. Distributeur implantable selon la revendication 1, caractérisé en ce que ledit moyen d'arrêt (19) situé entre lesdits moyens de propulsion (23) et le réservoir (4) comprend un diaphragme (20) qui porte de façon étanche contre une collerette (21) de l'élément de corps (1), et dans lequel ledit diaphragme (20) est traversé d'une ouverture (22).

18. Distributeur implantable selon la revendication 1, caractérisé en ce que lesdits moyens de propulsion (23, 33) comprennent un moyen à plongeur destiné à ouvrir et fermer, par un mouvement alternatif, une chambre (24, 34) de l'élément de corps en communication avec ledit canal (17) de raccordement.

19. Distributeur implantable selon la revendication 16, caractérisé en ce que lesdits moyens de propulsion (33) d'aval et lesdits moyens de propulsion (23) d'amont sont en communication de transmission de fluide par un segment dudit canal (17) de raccordement, de manière que l'enfoncement desdits moyens de propulsion (23) d'amont provoque une extension vers l'extérieur dudit moyen de propulsion (33) d'aval.

**_Fig_1_**

**_Fig_2_**

EP 0 143 503 B1

FIG_3

FIG_4